# EUROPEAN PATENT SPECIFICATION

(11) **EP 1 452 539 B1**
(45) Date of publication and mention of the grant of the patent: **06.01.2010**
(21) Application number: 02783582.6
(22) Date of filing: 21.11.2002
(51) Int. Cl.: C07K 14/46, A23J 1/04, C12N 15/09

(54) **ANTIFREEZE PROTEINS ORIGINATING IN FISH**
AUS FISCHEN STAMMENDE GEFRIERSCHUTZPROTEINE
PROTEINES ANTIGEL PROVENANT DE POISSONS

(30) Priority: 21.11.2001 JP 2001356709; 05.04.2002 JP 2002104477; 01.07.2002 JP 2002192339; 01.11.2002 JP 2002320425
(43) Date of publication of application: 01.09.2004
(73) Proprietor: National Institute of Advanced Industrial Science and Technology, Tokyo 100-8921 (JP)
(72) Inventor: TSUDA, Sakae, Sapporo-shi, Hokkaido 004-0021 (JP); MIURA, Ai, Sapporo-shi, Hokkaido 065-0021 (JP)
(74) Representative: Denison, Christopher Marcus
(86) International application number: PCT/JP2002/012153
(87) International publication number: WO 2003/044052

(56) References cited:
- WO-A1-97/02343
- US-A- 6 090 917
- MEINKE W W ET AL: "SOME FACTORS INFLUENCING THE PRODUCTION OF PROTEIN ISOLATES FROM WHOLE FISH" JOURNAL OF FOOD SCIENCE, XX, XX, vol. 37, 1972, pages 195-198, XP002915263
- SCHNEPPENHEIM R ET AL: "FREEZING-POINT DEPRESSING PEPTIDES AND GLYCOPROTEINS FROM ARCTIC-BOREAL AND ANTARCTIC FISH" POLAR BIOLOGY, SPRINGER VERLAG, DE, vol. 1, no. 2, 1 October 1982 (1982-10-01), pages 115-123, XP000575107 ISSN: 0722-4060
- WANG X. ET AL.: 'Antifreeze peptide heterogeneity in an antarctic eel pout includes an unusually large major variant comprised of two 7 kDa type III AFPs linked in tandem' BIOCHIM. BIOPHYS. ACTA vol. 1247, no. 2, 1995, pages 163 - 172, XP002236045
- WANG X, ET AL.: 'Genomic basis for antifreeze peptide heterogeneity and abundance in an antarctic eel pout: gene structures and organization' MOL. MAR. BIOL. BIOTECHNOL. vol. 4, no. 2, 1995, pages 135 - 147, XP009007178
- HEW C.L. ET AL.: 'Multiple genes provide the basis for antifreeze protein diversity and dosage in the ocean pout, macrozoarces americanus' J. BIOL. CHEM. vol. 263, no. 24, 1988, pages 12049 - 12055, XP002017638
- CHENG C,.H. ET AL.: 'Structures of antifreeze peptides from the antarctic eel pout, austrolycicthys branchycephalus' BIOCHIM. BIOPHYS. ACTA vol. 997, no. 1-2, 1989, pages 55 - 64, XP002980197

## Description

### FIELD OF THE INVENTION

The present invention relates to a protein obtained from fish inhabiting water areas in Japan, the peripheral water areas thereof, or water areas wherein the climate is equivalent to that of the above water areas, which has a function to inhibit freezing, and is obtained from antifreeze-protein-producing fish species belonging particularly to the genus *Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, Pholidapus*, or *Etrumeus*. More specifically, the present invention relates to an antifreeze protein obtained from fish species belonging to *Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Hypomesus pretiosus japonicus* (Brevoort), *Hypomesus olidus* (Dallas), *Spirinchus lanceolatus* (Hikita), *Mallotus villosus* (Muller), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Eopsetta grigorjewi* (Herzenstein), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), *Pholidapous dybowskii* (Steindachner), or *Etrumeus microps*.

### BACKGROUND OF THE INVENTION

A protein referred to as an antifreeze protein (abbreviated designation: AFP) has been discovered from the tissue fluid (blood) of fish caught at the Arctic Sea, the Antarctic Sea, and off the North American coast. This protein has a function of specifically binding to the surfaces of numerous small ice crystals generated in tissue fluid in a state immediately before freezing, deforming the crystals into a diamond shape, inhibiting freezing, and thus lowering the apparent freezing point of an aqueous solution containing the protein. Examples of such fish include *Gadus morhua Linneaeus* (English name: Atlantic cod), *Pseudopleuronectes americanus* (Walbaum) (English name: Winter flounder), *Clupea harengus Linnaeus* (English name: Atlantic herring), *Osmerus mordax dentex* (Steindachner) (English name: Rainbow smelt), *Myoxocephalus scorpius* (Linnaeus) (English name: Shorthorn Sculpin), *Macrozoarces americanus* (Block et Schneider), *Myoxocephalus octodecemspinosus* (Mitchill) (English name: Longhorn Sculpin), and *Hemitripterus americanus* (Gmelin) (English name: Athlantic sea raven) (Fletcher, G. L., Hew, C. L., and Davies, P. L. Annu. Rev. Physiol. (2001) 63, 359-390). As described above, antifreeze proteins have merely been discovered from a limited range of fish species. Besides, most of the above fish species are types of fish unavailable in Japan. It cannot be said that the presence of antifreeze proteins has been thoroughly searched for in the enormous number of fish species; that is, the 20,000 types or more of fish existing on earth.

In the meantime, antifreeze proteins are also known to be present in winter vegetables such as carrots, Japanese radishes, and cabbages, and the body fluids of the larvae of a beetle and a moth, in addition to fish. The antifreeze protein extracted from a plant has an ability to bind to ice crystals significantly weaker than that derived from fish. It is thought that since antifreeze proteins have a function of blocking the recrystallization of ice, when the protein is mixed into, for example, ice cream, it can preserve the quality. To block recrystallization more efficiently, an antifreeze protein having a stronger ability of binding to ice crystals should be mixed into such foods. With a plant-derived antifreeze protein having a weak ability of binding to ice crystals, such an effect cannot be expected. In contrast, a sufficient effect can be expected from a fish-derived antifreeze protein. However, most fish species that have been discovered to have antifreeze proteins to date are those inhabiting polar latitude areas or the arctic zone. Hence, it has been desired to isolate, purify, and utilize antifreeze proteins from fish species that are caught in large amounts in Japanese waters in a temperate zone area, or those marketed and discarded in large amounts from food super markets and the like.

Previous studies describing the purification of proteins from fish are of limited use in this respect. For example, Meinke, W. W., et al. [Journal of food Science (1972) vol.37:195-198] describes a method of preparing whole protein preparations from fish bodies. However, it does not describe a method of isolating a single protein or protein type. Schneppenheim, R., et al. [Polar Biology(1982) 1:115-123] describes a method for isolating the antifreeze protein FPDP from the skin and blood of Arctic-Boreal and Antarctic fish. However, this method was unable to purify antifreeze proteins from fish muscle tissue, making it of limited commercial use.

Moreover, when the purified product of an antifreeze protein derived from fish is used for frozen foods or the like, there is a need to prevent odors peculiar to fish from adhering to the purified product. However, studies on the storage and purification of fish bodies to meet this need have not shown progress.

### SUMMARY OF THE INVENTION

Hence, an object of the present invention is to discover a new antifreeze protein from fish species that are caught in large amounts in Japanese waters and the like, or marketed and discarded in large amounts from food super markets and the like, to produce an antifreeze protein in large quantities therefrom, and to promote the utilization of such antifreeze protein by preventing the adherence of fish odors.

Under such circumstances, we have intensively studied the body fluid components of fish species caught in Japan or in the periphery of Japan. Thus, we have discovered for the first time that some of fish species caught particularly in the winter season have antifreeze proteins, even if they are fish species caught in Japan or in the periphery of Japan, which is in a temperate zone area. We have confirmed the presence of a component that forms a diamond-shaped ice crystal in the tissue fluids and fluids prepared by mincing the muscles of various fish species for which the presence of antifreeze proteins has never been reported, for example, *Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Hypomesus pretiosus japonicus* (Brevoort), *Hypomesus olidus* (Dallas), *Spirinchus lanceolatus* (Hikita), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microstomus achne* (Jordan et Starks), *Lepidopsetta mochigarei* (Snyder), *Platichthys stellatus* (Pallas), *Kareius bicoloratus* (Basilewsky), *Eopsetta grigorjewi* (Herzenstein), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), and *Pholidapous dybowskii* (Steindachner), and in fluids prepared by adding water to crushed dry-fish-meat-products of, for example, *Theragra chalcogramma* (Pallas), *Eleginus gracilis, Spirinchus lanceolatus* (Hikita), *Mallotus villosus* (Muller), *Clupea pallasii* (Valenciennes), *Ammodytes personatus* (Girard), *Limanda aspera* (Pallas), and *Etrumeus microps*, which are sold as dry foods in food super markets and the like. Thus, we have completed the present invention. Above all, pond smelt (*Hypomesus*) is freshwater fish that has never before been reported to have an antifreeze protein generating a diamond-shaped ice crystal. Furthermore, we have addressed a major problem associated with fish-derived antifreeze protein, which is the adherence of fish odors, by establishing a new purification method.

That is, the present invention is as follows.
(1) A protein having a function to inhibit freezing, which is obtained from fish species inhabiting the water areas in Japan or the peripheral water areas of Japan, or water areas wherein the climate is equivalent to that of the above water areas.
(2) The protein of the above (1), wherein the source from which the protein is obtained is fish species belonging to the genus *Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, Pholidapus*, or *Etrumeus*.
(3) The protein of (2), wherein the source from which of the protein is obtained is fish species belonging to *Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Hypomesus pretiosus japonicus* (Brevoort), *Hypomesus olidus* (Dallas), *Spirinchus lanceolatus* (Hikita), *Mallotus villosus* (Muller), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Eopsetta grigorjewi* (Herzenstein), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), *Pholidapous dybowskii* (Steindachner), or *Etrumeus microps*.
(4) The protein of (1), which is obtained from the dry food composed of fish bodies.
(5) The protein of (2), which is obtained from the dry food composed of fish bodies.
(6) The protein of (3), which is obtained from the dry food composed of fish bodies.
(7) A method of collecting antifreeze proteins while removing the odors of fish bodies that are the source from which antifreeze proteins are obtained, which comprises the following steps 1) to 5):
   1) preparing a suspension of the paste composed of fish bodies or the crushed dry food composed of fish bodies;
   2) centrifuging the suspension of the paste composed of fish bodies or the crushed dry food composed of fish bodies obtained in 1) to obtain a supernatant solution;
   3) carrying out heat treatment for the supernatant solution obtained in 2);
   4) obtaining a supernatant solution containing anantifreeze protein by removing the precipitate generated in 3) by centrifugation; and
   5) collecting the antifreeze proteins from the supernatant solution obtained in 4).
(8) A protein of the following (a) or (b):
   (a) a protein, comprising an amino acid sequence represented by SEQ ID NO: 1, or
   (b) a protein, comprising an amino acid sequence derived from the amino acid sequence represented by SEQ ID NO: 1 by deletion, substitution, or addition of 1 or several amino acids, and having a function to inhibit freezing.
(9) Anantifreeze protein gene encoding the following protein (a) or (b):
   (a) a protein comprising the amino acid sequence represented by SEQ ID NO: 1, or
   (b) a protein comprising an amino acid sequence derived from the amino acid sequence represented by SEQ ID NO: 1 by deletion, substitution, or addition of 1 or several amino acids and having a function to inhibit freezing.
(10) A gene comprising the following DNA (a) or (b):
   (a) a DNA comprising a nucleotide sequence represented by SEQ ID NO: 2; or
   (b) a DNA hybridizing under stringent conditions to a DNA comprising a nucleotide sequence complementary to a DNA comprising the whole or a part of the nucleotide sequence represented by SEQ ID NO: 2 and encoding a protein having a function to inhibit freezing.
(11) A protein of the following (a) or (b):
   (a) a protein comprising an amino acid sequence represented by SEQ ID NO: 3; or
   (b) a protein comprising an amino acid sequence derived from the amino acid sequence represented by SEQ ID NO: 3 by deletion, substitution, or addition of 1 or several amino acids and having a function to inhibit freezing.
(12) An antifreeze protein gene encoding the following protein (a) or (b):
   (a) a protein comprising an amino acid sequence represented by SEQ ID NO: 3; or
   (b) a protein comprising an amino acid sequence derived from the amino acid sequence represented by SEQ ID NO: 3 by deletion, substitution, or addition of 1 or several amino acids and having a function to inhibit freezing.
(13) A gene comprising the following DNA (a) or (b):
   (a) a DNA comprising a nucleotide sequence represented by SEQ ID NO: 4; or
   (b) a DNA hybridizing under stringent conditions to a DNA comprising a nucleotide sequence complementary to a DNA comprising the whole or a part of the nucleotide sequence represented by SEQ ID NO: 4 and encoding a protein having a function to inhibit freezing.
(14) A protein of the following (a) or (b):
   (a) a protein comprising an amino acid sequence represented by SEQ ID NO: 5; or
   (b) a protein comprising an amino acid sequence derived from the amino acid sequence represented by SEQ ID NO: 5 by deletion, substitution, or addition of 1 or several amino acids and having a function to inhibit freezing.
(15) An antifreeze protein gene encoding the following protein (a) or (b):
   (a) a protein comprising an amino acid sequence represented by SEQ ID NO: 5; or
   (b) a protein comprising an amino acid sequence derived from the amino acid sequence represented by SEQ ID NO: 5 by deletion, substitution, or addition of 1 or several amino acids and having a function to inhibit freezing.
(16) A gene comprising the following DNA (a) or (b):
   (a) a DNA comprising a nucleotide sequence represented by SEQ ID NO: 6; or
   (b) a DNA hybridizing under stringent conditions to a DNA comprising a nucleotide sequence complementary to a DNA comprising the whole or a part of the nucleotide sequence represented by SEQ ID NO: 6, and encoding a protein having a function to inhibit freezing.

The present invention will be described in detail as follows.

The body fluids of fish inhabiting the polar regions and the deep seas of the north pole, the south pole, or seas surrounding such regions do not freeze when the temperature falls to approximately - 2°C. In contrast, the body fluids of general fish freeze at - 0.8°C. In addition, seawater freezes at - 1.9°C.

The anti-freezing ability of the body fluids of fish inhabiting the north pole, the south pole, or the like has been shown to be caused by anantifreeze protein or an antifreeze glycoprotein (AFGP) that is a fish's own product. There are 4 types of antifreeze proteins, and these are classified as AFPI with an approximate molecular weight between 3,000 and 4,500, AFPII with an approximate molecular weight of 20,000, AFPIII with an approximate molecular weight of 7,000, and AFPIV with an approximate molecular weight of 11,000. The molecular weight of an AFGP is between 2,200 and 33,000. Whether or not the purified protein is an AFGP can be easily confirmed using Schiffs reagent or the like. Each antifreeze protein has a different amino acid composition, and a different higher-order structure. Even if antifreeze proteins are classified as being of the same type, they may differ in their amino acid sequences and higher-order structures, depending on fish species.

Furthermore, the functions of an antifreeze protein are explained. In a general case, an ice crystal first grows into the shape of flat hexagonal plate when a nucleus of ice appears in an aqueous solution. The ice grows perpendicular to the flat plate approximately 100 times slower than growth in the direction of the flat plate. In contrast, the presence of antifreeze proteins in an aqueous solution blocks the growth of ice crystals in the direction of the plane surface of a disk. Specifically, a smaller plate-shaped body is subsequently stacked perpendicular to a basal layer, which is the plate-shaped body formed at the beginning, and then finally slowly grows into a bi-pyramid-type ice crystal (bi-pyramidal ice crystal) formed of two stacked pyramids.

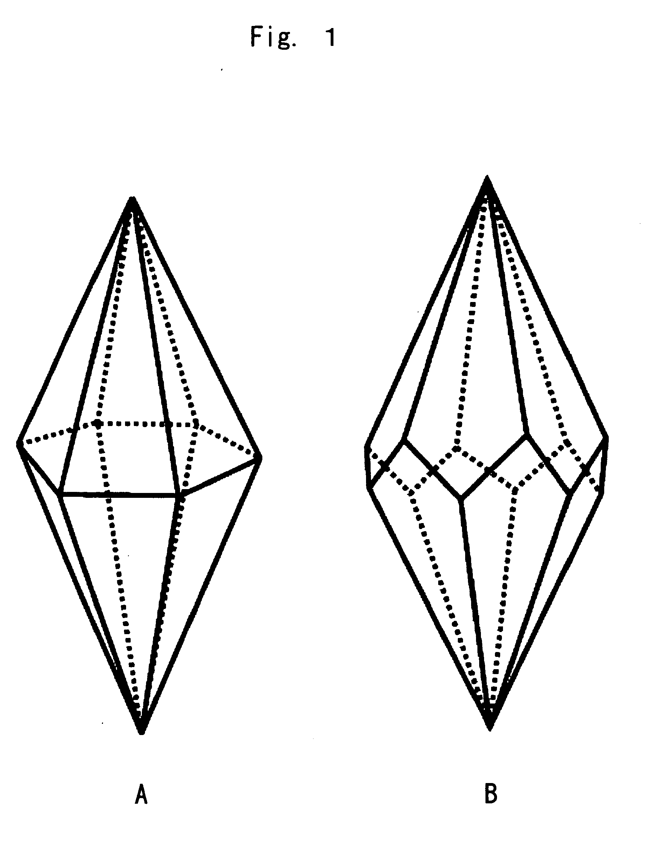

Hence, it is only when antifreeze proteins are present in an aqueous solution obtained from samples of fish species of interest, and when the temperature of sample solutions are kept at 0°C or less, that in the sample solutions, bi-pyramidal ice crystals as shown in Fig 1A and B are observed. Crystallographically, the ice crystals with shapes referred to as "dihexahedrons" (Fig. 1A) and "dodecahedron having irregular tetragons" (Fig. 1B) can be observed under a microscope. As a result of the ability of antifreeze proteins to specifically bind to 12 ice layer surfaces of an ice crystal, such bi-pyramidal ice crystals are generated. In the freezing temperature range at 0°C or less, microscopically, antifreeze proteins in a sample solution generate numerous bi-pyramidal ice crystals that do not bind to each other. This is macroscopically observed as a phenomenon where a sample does not freeze (antifreeze activity). This phenomenon can also be quantified as a resulting decreased freezing point of a sample solution or temperature hysteresis using an osmometer. An aqueous solution of purified antifreeze proteins with a high degree of purity is required in order to find the presence of antifreeze proteins using a method for measuring decreases in freezing point, and in order to evaluate such activity. On the other hand, in a method for evaluating antifreeze activity by observing bi-pyramidal ice crystals, with the presence of only an antifreeze protein, bi-pyramidal ice crystals are observed even when impurities, contaminating proteins, ions, and the like coexist in large quantities in the sample solution. In addition, when the concentration of antifreeze proteins is 0.1 mM or more, bi-pyramidal ice crystals are observed regardless of the concentration. Hence, it can be said that the most convenient and rapid technique to evaluate whether or not a sample, that is, a fish species of interest has antifreeze proteins is to observe bi-pyramidal ice crystals in a sample solution.

In all the fish species, the sources from which the antifreeze protein of the present invention is obtained, the presence of bi-pyramidal ice crystals has been confirmed in the sample solutions prepared from the blood, fish paste, or dry foods thereof at freezing temperatures.

It is extremely significant that antifreeze proteins have been discovered in the present invention from various fish species inhabiting water areas in Japan and the peripheral water areas thereof. Based on this result, it can be said that with an extremely high probability, fish species capable of synthesizing *in vivo* antifreeze proteins are present among fish inhabiting the water areas anywhere in the Northern and the Southern Hemispheres, and substantially water areas wherein the climate is at least equivalent to that of the above water areas. The fishery time for the fish species of the present invention is preferably from the end of October to the beginning of May, and in particularly preferably from December in winter season to the beginning of April. Antifreeze proteins can be obtained from the blood, fish paste, and the aqueous extract of dry foods of the fish species of the present invention by carrying out an appropriate combination of general means for separating and purifying proteins, such as ion exchange chromatography, gel filtration chromatography, and reverse phase chromatography.

In general, it is thought that in most cases, proteins in a fish body are degraded by enzymes and the like immediately after the death of the fish body, so as to lose their ability. Hence, it is surprising that antifreeze proteins can also be purified from fish bodies long after the death, such as at the time when they are sold in food super markets, and the like. In the present invention, a finding of the fact that antifreeze proteins can be sufficiently purified from fish bodies that are sold in food super markets and the like, and a finding of the fact that antifreeze proteins can be purified even from the dry food composed of fish bodies that have been heated, washed, dried, and the like can also be said to be extremely significant. These facts are based on the incredibly high stability of fish-derived antifreeze proteins. Because of this rare property, in a step of purifying antifreeze proteins from a suspension of minced fish bodies or crushed dry foods composed of fish bodies, heat treatment at a high temperature can be applied.

Unpleasant odors coming from fish are due to the decay of organs in the head and abdominal portions and the content thereof as time passes, and fish meat's own unique odor. Therefore, early removal of the head portion and the abdominal portion from fish bodies to be used as raw materials for the purification of antifreeze proteins is basically preferable. After the removal of the abdominal and the head portions, fish bodies can be stored after drying, in addition to being stored by refrigeration. This drying has an effect of concentrating antifreeze proteins, or an effect of suppressing the proliferation of bacteria. Drying is also beneficial in terms of reducing the weight of fish bodies upon storage and the like. Moreover, drying is also beneficial in terms of preservation, transport, overseas export, and the like, because it enables storage at room temperature.

In the present invention, antifreeze proteins may also be obtained by applying the above generally used method for separating and purifying proteins. To obtain an antifreeze protein having no fish odors and being appropriate as a food additive, it is effective to use a method for purifying antifreeze proteins, comprising the steps in order of: 1) preparing a suspension of minced fish bodies or the crushed dry food composed of fish bodies; 2) centrifuging the suspension of minced fish bodies or of the crushed dry food composed of fish bodies obtained in 1) to obtain a supernatant solution; 3) carrying out heat treatment for the supernatant solution obtained in 2); 4) obtaining a supernatant solution containing antifreeze proteins by removing the precipitate generated in 3) by centrifugation; and 5) collecting the antifreeze proteins from the supernatant solution obtained in 4).

Specifically, in step 1), fish meat is minced, or the dry food composed of fish bodies is cut into small pieces with scissors or the like, followed by crushing with a mixer or the like. To the paste or the crushed product, water or an aqueous solution of such as ammonium bicarbonate or sodium hydrogen phosphate is added to prepare a suspension of fish meat. Hence, antifreeze proteins are eluted in the aqueous solution. Fish paste may be obtained by cutting fish meat into small pieces and then applying the pieces to a mixer, and may also be obtained by mincing fish meat using a paste-making machine according to a conventional method. In the step 2), the above paste suspension or the suspension of the crushed dry food composed of fish bodies is centrifuged, thereby obtaining a supernatant solution containing antifreeze proteins. Centrifugation conditions consist of 3,000 to 12,000 revolutions/minute for 5 to 60 minutes.

Subsequently, in step 3), the supernatant solution obtained in the above step is treated with heat. Heat treatment is carried out for effectively removing or reducing the odors unique to fish bodies and to fish paste, as well as causing thermal denaturation of contaminating proteins other than antifreeze proteins so as to cause such proteins to precipitate. Accordingly, it can be said that higher temperatures within a temperature range of 100°C or less are preferably set upon heat treatment, as long as the temperature does not cause denaturation or precipitation of antifreeze proteins, the target purification product. For the suspensions of the paste of *Myoxocephalus stelleri* (Tilesius), *Myoxocephalus polyacanthocephalus* (Pallas), *Gymnocanthus herzensteini* (Jordan et Starks), *Hemilepidotus jordani* (Bean), and *Furcina osimae* (Jordan et Starks), whose antifreeze proteins are extremely resistant to heat, heat treatment at 90°C was carried out for 10 minutes, so that an odor-free antifreeze protein component could be obtained. When these fish species are used, heat treatment within a temperature range between 70°C and 98°C for 10 minutes to 30 minutes is preferred.

On the other hand, for Hypomesus pretiosus japonicus (Brevoort), Hypomesus olidus (Dallas), Spirinchus lanceolatus (Hikita), Pleurogrammus monopterygius (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microstomus achne* (Jordan et Starks), *Lepidopsetta mochigarei* (Snyder), *Platichthys stellatus* (Pallas), *Kareius bicoloratus* (Basilewsky), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), and *Pholidapous dybowskii* (Steindachner), the step of heat treatment is carried out within the range between 50°C and 70°C for 10 to 30 minutes. With this temperature range, purified specimens of antifreeze proteins with purities of 60% or more, from which fish odors had been removed, could be obtained. Purified specimens of antifreeze proteins with such purities can be said to have quality sufficient for some applications.

In step 4), the supernatant solution subjected to the above heat treatment is centrifuged, and then the precipitated contaminating proteins are removed. The obtained supernatant solution containing antifreeze proteins at a high concentration may be used in its intact form for applications described later, and is preferably processed into dry powder by freeze-drying.

The amino acid sequences of the antifreeze proteins of the present invention are illustrated in SEQ ID NOS: 1, 3 and 5, and the nucleotide sequences of the antifreeze protein genes of the present invention corresponding to these amino acid sequences are illustrated in SEQ ID NOS: 2, 4 and 6, respectively. As long as the proteins comprising these amino acid sequences have a function to inhibit freezing, mutations such as deletion, substitution, and addition of 1 or several amino acids may occur in the amino acid sequences.

For example, 1 amino acid, preferably 10 to 20 amino acids, and more preferably 5 to 10 amino acids may be deleted from the amino acid sequence represented by SEQ ID NO: 1; 1 amino acid, preferably 10 to 20 amino acids, and more preferably 5 to 10 amino acids may be added to the amino acid sequence represented by SEQ ID NO: 1; or 1 amino acid, preferably 10 to 20 amino acids, and more preferably 5 to 10 amino acids may be substituted with other amino acids in the amino acid sequence represented by SEQ ID NO: 1. Moreover, the gene of the present invention also includes a gene hybridizing under stringent conditions to a sequence complementary to a DNA comprising the whole or a part of the nucleotide sequence of the DNA comprising the nucleotide sequence of the antifreeze protein gene of the present invention, and encoding a protein having a function to inhibit freezing. Here, the stringent conditions refer to conditions where specific hybrids are formed but non-specific hybrids are not formed. Specifically, it refers to conditions where a DNA having high homology (homology of 90% or more, and preferably 95% or more) to the antifreeze protein gene of the present invention hybridizes. More specifically, such conditions can be achieved by carrying out hybridization in the presence of 0.5 to 1 M NaCl at 42°C to 68°C, in the presence of 50% formamide at 42°C, or in an aqueous solution at 65°C to 68°C, and then washing the filter using a 0.1 to 2-fold concentration of SSC (saline sodium citrate) solution at room temperature to 68°C.

Here, "a part of a sequence" refers to a nucleotide sequence of a DNA containing a part of the nucleotide sequence of the above antifreeze protein gene, and encoding a protein having a function to inhibit freezing. Furthermore, "a part of a sequence" refers to a sequence having a nucleotide sequence length sufficient for hybridization under stringent conditions, for example, a sequence of at least 10 nucleotides, preferably at least 50 nucleotides, and more preferably at least 200 nucleotides.

As described above, antifreeze proteins have action of blocking recrystallization of ice. Thus, the antifreeze protein of the present invention obtained from each of the above fish species can be used as an agent for preventing recrystallization of ice or as a freeze inhibitor regardless of which type (AFP I to IV) the antifreeze protein is classified as. For example, when the antifreeze protein is mixed into ice cream, frozen foods, or the like, the quality of the foods can be maintained. Moreover, the effect of maintaining quality can be similarly expected for long term cryopreservation of meat, vegetables, cells (egg cells and sperm), tissues, organs, and the like. Furthermore, recently, a cold heat supply system, cold heat storage, and the like using ice slurries having large energy density as a heat medium have been proposed. However, these systems are problematic in that their piping systems become blocked by the recrystallization of ice. The antifreeze protein of the present invention effectively prevents recrystallization of ice, and so can be a promising means to address this problem. Moreover, conferring cold resistance to a plant by incorporation of a gene encoding the antifreeze protein into a plant body can also be expected as an applied technology.

### BRIEF DESCRIPTION OF THE DRAWINGS

Fig. 1 is a schematic view showing the shape of bi-pyramidal ice crystals.
Fig. 2 shows microphotographs of bi-pyramidal ice crystals observed in the blood of *Myoxocephalus stelleri* (Tilesius), *Myoxocephalus polyacanthocephalus* (Pallas), *Gymnocanthus herzensteini* (Jordan et Starks), *Hypomesus pretiosus japonicus* (Brevoort), *Hypomesus olidus* (Dallas), and *Zoarces elongatus* (Kner).
Fig. 3 shows microphotographs of bi-pyramidal ice crystals observed in the blood of *Limanda herzensteini* (Jordan et Snyder), *Liopsetta obscura* (Herzenstein), *Limanda punctatissima* (Steindachner), *Lepidopsetta mochigarei* (Snyder), *Clidoderma asperrimum* (Temminck et Schlegel), and *Cleisthenes pinetorum herzensteini* (Schmidt).
Fig. 4 shows microphotographs of bi-pyramidal ice crystals observed in the blood or suspensions of the paste of *Theragra chalcogramma* (Pallas), *Pleurogrammus monopterygius* (Pallas), *Sebastes steindachneri* (Hilgendort), *Clupea pallasii* (Valenciennes), *Spirinchus lanceolatus* (Hikita), and *Limanda schrenki* (Schmidt).
Fig. 5 shows microphotographs of bi-pyramidal ice crystals observed in the blood or suspensions of the paste of *Platichthys stellatus* (Pallas), *Limanda aspera* (Pallas), *Kareius bicoloratus* (Basilewsky), *Furcina osimae* (Jordan et Starks), *Ammodytes personatus* (Girard), and *Pholidapous dybowskii* (Steindachner).
Fig. 6 shows microphotographs of bi-pyramidal ice crystals observed in the blood or suspensions of the paste of *Gadus macrocephalus* (Tilesius), *Microstomus achne* (Jordan et Starks), *Hemilepidotus jordani* (Bean), *Ascoldia variegata knipowitschi* (Soldatov), *Hypoptychus dybowskii* (Steindachner), and *Eopsetta grigorjewi* (Herzenstein).
Fig. 7 shows microphotographs of bi-pyramidal ice crystals observed in suspensions of the paste of *Pholis picta* (Kner), *Opisthocentrus ocellatus* (Tilesius), *Brachyopsis rostratus* (Tilesius), and *Mallotus villosus* (Muller).
Fig. 8 shows microphotographs of bi-pyramidal ice crystals observed in suspensions of crushed dry foods of *Etrumeus microps, Trachurus japonicus* (Temminck et Schlegel), *Theragra chalcogramma* (Pallas), *Spirinchus lanceolatus* (Hikita), and *Limanda aspera* (Pallas).
Fig. 9 shows absorbance patterns observed when 280-nm ultraviolet rays were irradiated to gel filtration fractions of *Myoxocephalus stelleri* (Tilesius) serum.
Fig. 10 shows elution patterns of reverse phase HPLC chromatography for the above gel filtration fraction numbers 10 to 20.
Fig. 11 shows the results of SDS gel electrophoresis carried out for fractions AFP1 and AFP2 obtained by this reverse phase HPLC chromatography.
Fig. 12 shows A: the results of SDS gel electrophoresis carried out for the centrifugal supernatant of suspensions of the paste of *Hypomesus pretiosus japonicus* (Brevoort) before and after heat treatment at 70°C for 10 minutes; and B: the elution patterns of gel chromatography carried out for a sample solution after heat treatment at 70°C for 10 minutes.

This specification includes part or all of the contents as disclosed in the specifications of Japanese Application Nos. 2001-356709, 2002-104477, 2002-192339, and 2002-320425, which are priority document of the present application.

### BEST MODE FOR CARRYING OUT THE INVENTION

Examples of the present invention will be shown below, but the present invention is not specifically limited by these examples.

### Example 1

### (Confirmation of the presence of antifreeze protein)

### (1) Sample specimen

The following fish species were used as the sources from which antifreeze proteins were obtained.

*Myoxocephalus stelleri* (Tilesius); English name: Frog Sculpin: caught off the coast of the Rishiri Island (*Rishiri-to*).

*Gymnocanthus herzensteini* (Jordan et Starks); English name: Black edged sculpin: caught off the coast of the Rishiri Island (*Rishiri-to*).

*Myoxocephalus polyacanthocephalus* (Pallas); English name: Great Sculpin: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido) and the Hokkaido Notsuke fishermen's cooperative association (*Hokkaido Notsuke Gyogyo Kyodo Kumiai*).

*Hypomesus pretiosus japonicus* (Brevoort): purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido) and the Hokkaido Notsuke fishermen's cooperative association (*Hokkaido Notsuke Gyogyo Kyodo Kumiai*).

*Zoarces elongatus* (Kner); English name: Notched-fin eelpout: purchased at the Hokkaido Notsuke fishermen's cooperative association (*Hokkaido Notsuke Gyogyo Kyodo Kumiai*).

*Hypomesus olidus* (Dallas); English name: Freshwater smelt: purchased at the Barato-gawa Smelt (*Hypomesus olidus*) fishing spot (Barato Ko-en, Kita-ku, Sapporo-shi). *Limanda herzensteini* (Jordan et Snyder); English name: Brown Sole: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Limanda punctatissima* (Steindachner); English name: Longsnout flounder: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Liopsetta obscura* (Herzenstein); English name: Black plaice: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Clidodema asperrimum* (Temminck et Schlegel); English name: Roughscale sole: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Cleisthenes pinetorum herzensteini* (Schmidt); English name: Pointhead flounder: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Microstomus achne* (Jordan et Starks); English name: Slime flounder: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Lepidopsetta mochigarei* (Snyder); English name: Dusky Sole: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Eopsetta grigorjewi* (Herzenstein); English name: Shothole halibut: caught off the coast of Ishikari Bay, Hokkaido.

*Gadus macrocephalus* (Tilesius); English name: Pacific cod: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Hemilepidotus jordani* (Bean); English name: Yellow Sculpin: caught off the coast of Samani-cho, Hokkaido.

*Hypoptychus dybowskii* (Steindachner); English name: Naked sand lance: caught off the coast of the Rishiri island (*Rishiri-to*), Hokkaido.

*Ascoldia variegata knipowitschi* (Soldatov): caught off the coast of Samani-cho, Hokkaido.

*Theragra chalcogramma* (Pallas); English name: Walleye Pollock: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Pleurogrammus monopterygius* (Pallas); English name: Atka mackerel: caught off the coast of Ishikari Bay, Hokkaido.

*Sebastes steindachneri* (Hilgendorf): caught off the coast of Ishikari Bay, Hokkaido. *Clupea pallasii* (Valenciennes); English name: California herring: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Spirinchus lanceolatus* (Hikita); English name: Sishamo Smelt: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido), and caught off the coast of Mukawa-cho, Hokkaido.

*Limanda schrenki* (Schmidt); English name: Cresthead flounder: caught off the coast of Ishikari Bay, Hokkaido.

*Platichthys stellatus* (Pallas); English name: Starry flounder: caught off the coast of Ishikari Bay, Hokkaido.

*Limanda aspera* (Pallas); English name: Yellow fin sole: caught off the coast of Ishikari Bay, Hokkaido.

*Kareius bicoloratus* (Basilewsky); English name: Stone flounder: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido), and caught at the coast of Ishikari Bay, Hokkaido.

*Furcina osimae* (Jordan et Starks); English name: Silk Sculpin): caught off the coast of Tomakomai, Hokkaido.

*Ammodytes personatus* (Girard); English name: Japanese sand lance: caught off the coast of Ishikari Bay, Hokkaido.

*Pholidapous dybowskii* (Steindachner); English name: Dybowsky's gunnel: caught off the coast of Ishikari Bay, Hokkaido.

*Brachyopsis rostratus* (Tilesius); English name: Longsnout poacher: caught in Notsuke Bay, Hokkaido.

*Pholis picta* (Kner); English name: Painted gunnel: caught in Notsuke Bay, Hokkaido. *Opisthocentrus ocellatus* (Tilesius); English name: Redspotted gunnel: caught in Notsuke Bay, Hokkaido.

*Mallotus villosus* (Muller); English name: Atlantic capelin: purchased at Fujitomi Suisan (Atsubetsu I Cowboy, Atsubetsu Nishi, 4-jyo, 2-chome, 752-banchi, 318, Atsubetsu-ku, Sapporo-shi, Hokkaido).

*Trachurus japonicus* (Temminck et Schlegel); English name: Yellowfin horse mackerel: purchased at *Fujitomi Suisan* (Atsubetsu I Cowboy, Atsubetsu Nishi, 4-jyo, 2-chome, 752-banchi, 318, Atsubetsu-ku, Sapporo-shi, Hokkaido).

Dry foods composed of fish bodies used as the sources from which antifreeze proteins are obtained are as shown below.

*Theragra chalcogramma* (Pallas); Brand name: *Mushiri-tara*, Produced by: DAITO FOODS CO., LTD.: purchased at Atsubetsu I Cowboy, Atsubetsu Nishi, 4-jyo, 2-chome, 752-banchi, 318, Atsubetsu-ku, Sapporo-shi, Hokkaido.

*Spirinchus lanceolatus* (Hikita); Brand name: *Kokunai-san Hime Shishamo*, Produced by Daimaru Suisan: purchased at Atsubetsu I Cowboy, Atsubetsu Nishi, 4-jyo, 2-chome, 752-banchi, 318, Atsubetsu-ku, Sapporo-shi, Hokkaido.

*Ammodytes personatus* (Girard); Brand name: *Oonago Kunsei*; Produced by Fujimizu: purchased at Atsubetsu I Cowboy, Atsubetsu Nishi, 4-jyo, 2-chome, 752-banchi, 318, Atsubetsu-ku, Sapporo-shi, Hokkaido.

*Limanda aspera* (Pallas); Brand name: *Roll Kogane Garei*; Produced by DAITO FOODS CO., LTD.: purchased at Atsubetsu I Cowboy, Atsubetsu Nishi, 4-jyo, 2-chome, 752-banchi, 318, Atsubetsu-ku, Sapporo-shi, Hokkaido.

*Etrumeus microps*; Brand name: *Urume Niboshi*; Produced by KANESHITI Co., Ltd.: purchased at Atsubetsu I Cowboy, Atsubetsu Nishi, 4-jyo, 2-chome, 752-banchi, 318, Atsubetsu-ku, Sapporo-shi, Hokkaido.

### (2) Observation of bi-pyramidal ice crystals

### a) The branchial portion of fresh fish of each of the above fish species was prized open.

While holding the fish body with a spoon or the like so as not to damage the organs such as the liver and the small intestine, an injection needle attached to a syringe (capacity: 5 ml) was carefully inserted into the triangular dark red heart portion that could be seen on the head portion side, so as to collect blood. The total volume of blood collected was 150 ml in each fish species of Myoxocephalus stelleri (Tilesius), Gymnocanthus herzensteini (Jordan et Starks), and Hypomesus pretiosus japonicus (Brevoort), 420 ml in the fish species of *Myoxocephalus polyacanthocephalus* (Pallas), and 30 ml in each fish species of *Pleurogrammus monopterygius* (Pallas) and *Clupea pallasii* (Valenciennes). Moreover, the total volume of blood collected was 20 ml in each fish species of *Limanda herzensteini* (Jordan et Snyder), *Limanda punctatissima* (Steindachner), *Liopsetta obscura* (Herzenstein), *Clidoderma asperrimum* (Temminck et Schlegel), *Microstomus achne* (Jordan et Starks), *Lepidopsetta mochigarei* (Snyder), *Limanda schrenki* (Schmidt), *Platichthys stellatus* (Pallas), *Limanda aspera* (Pallas), *Kareius bicoloratus* (Basilewsky), and *Eopsetta grigorjewi* (Herzenstein), 35 ml in each fish species of *Gadus macrocephalus* (Tilesius) and *Theragra chalcogramma* (Pallas), and 25 ml in each fish species of *Hemilepidotus jordani* (Bean), *Furcina osimae* (Jordan et Starks), *Zoarces elongatus* (Kner), *Ascoldia variegata knipowitschi* (Soldatov), and *Pholidapous dybowskii* (Steindachner). These blood samples were used in the following (b) and an experiment in Example 2. For fresh fish other than the above fish, such as *Hypomesus olidus* (Dallas), *Sebastes steindachneri* (Hilgendorf), *Cleisthenes pinetorum herzensteini* (Schmidt), *Spirinchus lanceolatus* (Hikita), *Mallotus villosus* (Muller), *Hypoptychus dybowskii* (Steindachner), *Ammodytes personatus* (Girard), *Brachyopsis rostratus* (Tilesius), *Pholis picta* (Kner), and *Opisthocentrus ocellatus* (Tilesius), fish meat portions excluding skin, bones, and organs were excised using a kitchen knife, cut finely, and then pulverized with a mixer, thereby preparing fish paste. An equivalent volume (20 ml) of 0.1 M ammonium sulfate aqueous solution (pH 7.9) was added per 20 ml of fish paste to prepare a suspension. Furthermore, dry foods of the fish bodies of *Theragra chalcogramma* (Pallas), *Spirinchus lanceolatus* (Hikita), *Ammodytes personatus* (Girard), *Limanda aspera* (Pallas), *Etrumeus microps*, and *Trachurus japonicus* (Temminck et Schlegel) were cut into approximately 1 cm pieces with scissors, and then pulverized using a mixer. 20 ml of a 0.1 M ammonium sulfate aqueous solution (pH=7.9) was added per 20 g of the pulverized product. The solution was mixed well, and then allowed to stand overnight at 4°C, so as to prepare a suspension of the dry foods. The thus obtained blood, the suspension of fish paste, and the suspension of dry foods were used in the following (b) and the experiment in Example 2.

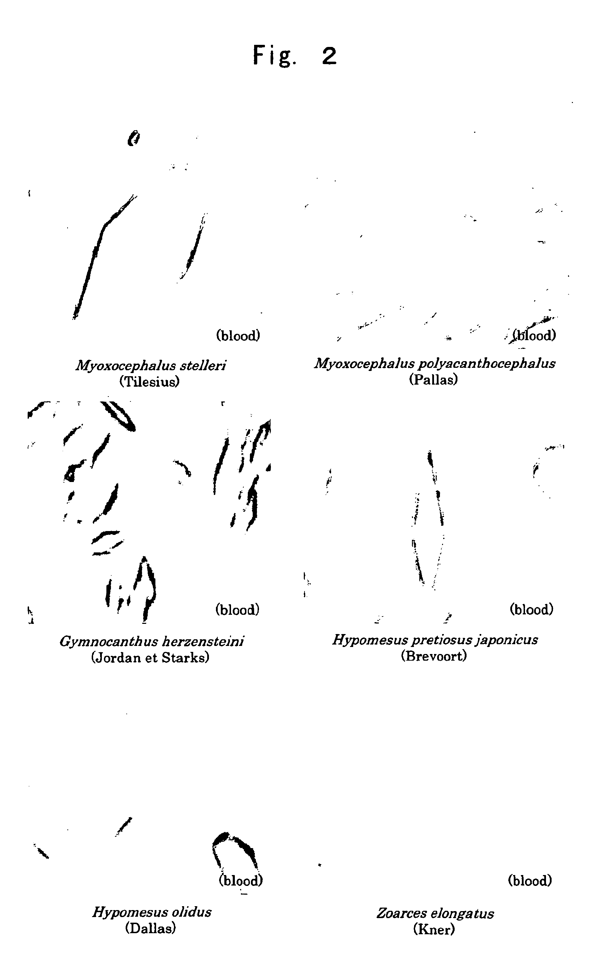

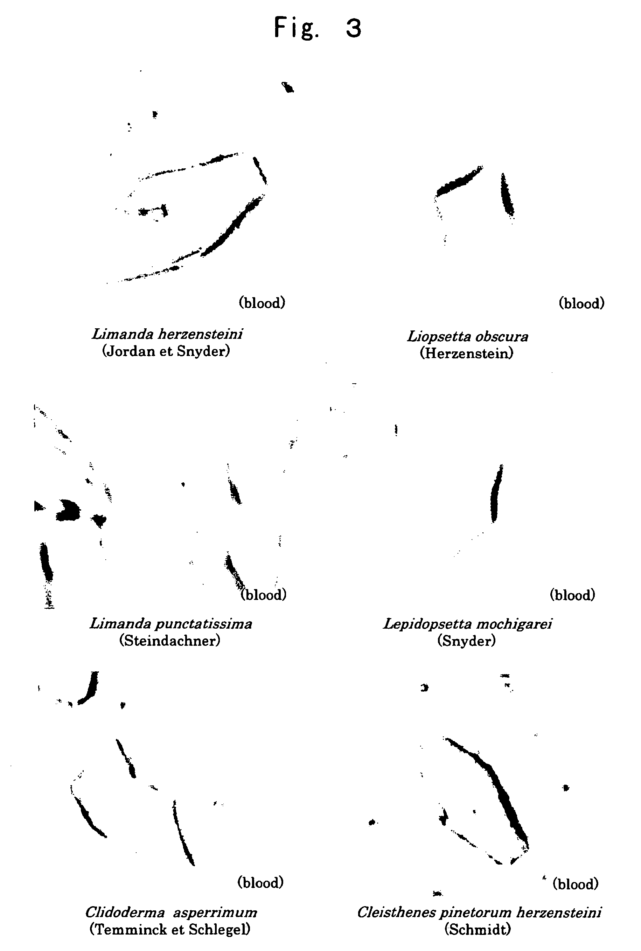

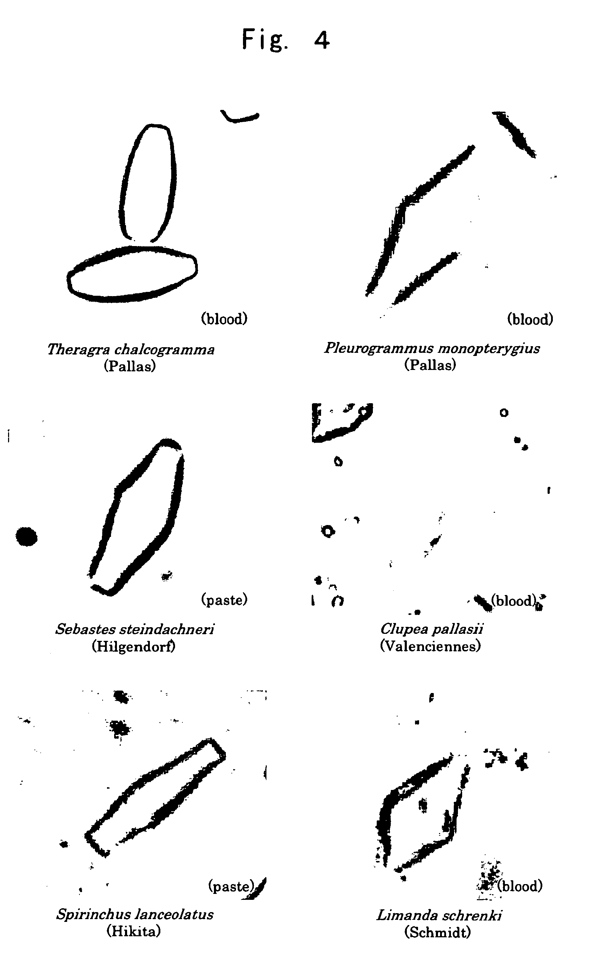

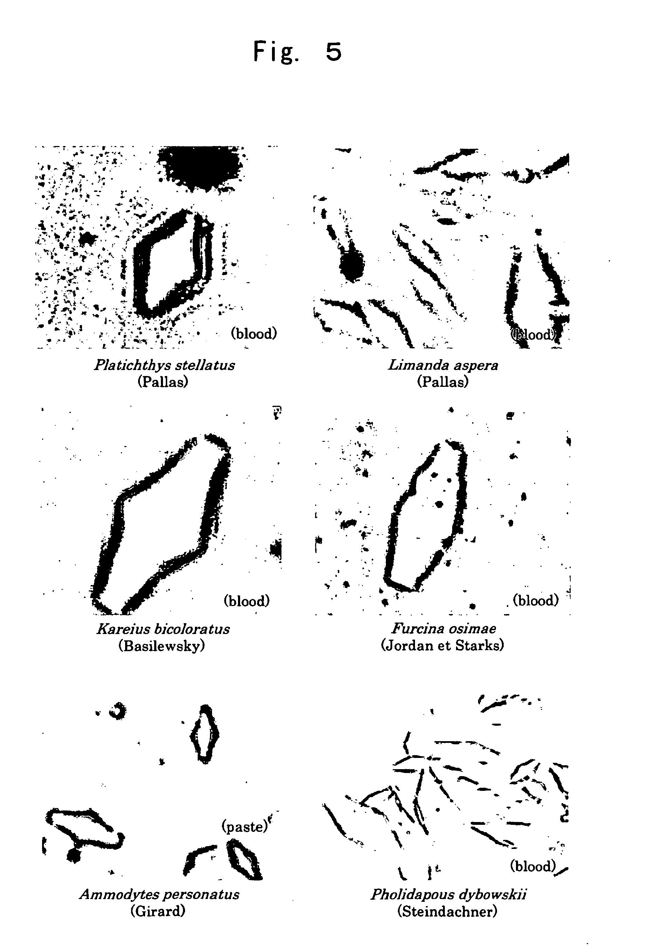

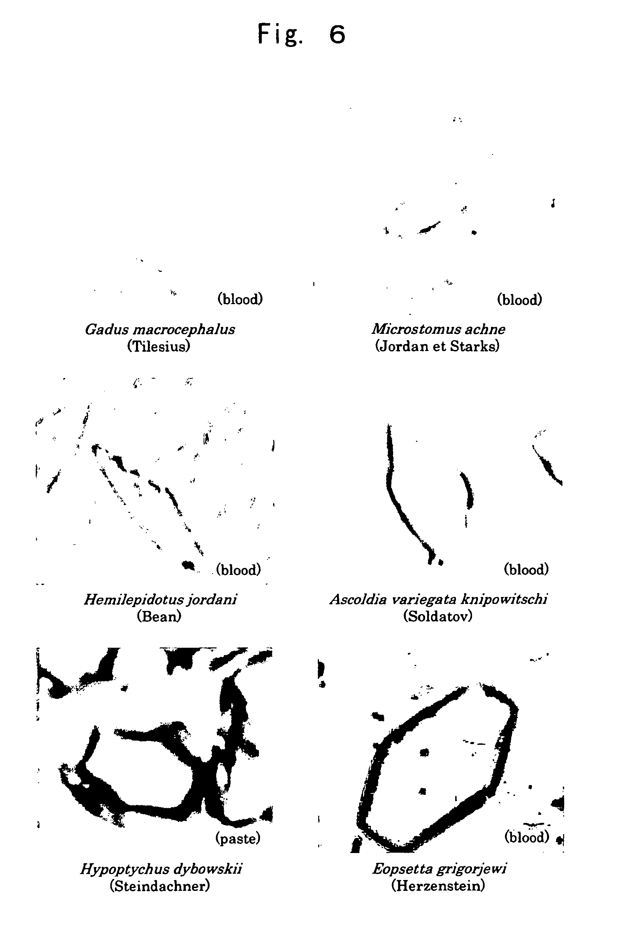

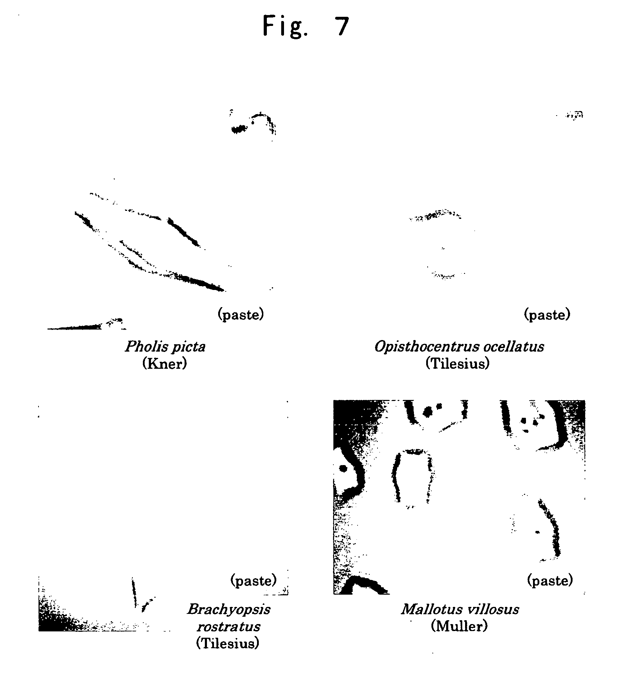

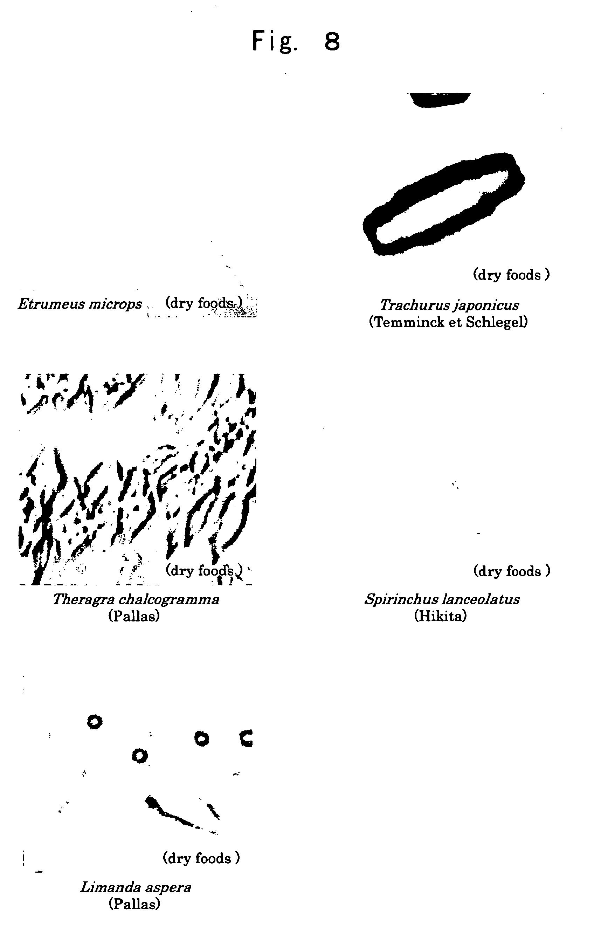

Whether or not a sample solution of interest contains an antifreeze protein can be evaluated by carrying out an experiment observing bi-pyramidal ice crystals under a low temperature microscope using only 1 µl of the solution. b) 1 µl of blood of each fish species collected as described above was placed dropwise on a cover glass with a diameter of 16 mm of a DMLB100 photomicroscope (manufactured by Leica Microsystems AG). The sample was placed between this cover glass and another cover glass with a diameter of 12.5 mm, and set in a cooler box provided at the stage portion of the DMLB100 photomicroscope. Holes with a diameter of 1 mm were made on the top and on the bottom of the cooler box for lightning, so that light from the light source of the photomicroscope entered the hole on the bottom, passed through the inside of the box and the hole on the top, and thus entered the lens. The sample solution was set on the optical axis specified by the holes on the top and on the bottom, so that substances in the sample solution on the optical axis could be observed under a microscope. The temperature within the cooler box wherein the sample solution was set was controlled by an LK600 temperature controller (manufactured by Linkam) with an error range of +/-0.1°C. When the sample solution was set at room temperature, the temperature within the cooler box was lowered by 0.2°C every second to -22°C using the temperature controller. The entire sample solution froze at temperatures somewhere between approximately -1.4°C and -2.2°C. After freezing, the temperature within the cooler box was raised by 0.1°C every second, and this was stopped at 0°C. When the temperature was maintained at 0°C for approximately 1 to 10 seconds, the frozen solution started to defreeze, and passed through the ice crystal state with numerous cracks. Then, an approximately countable number of grains of ice crystals floating in water were observed. At this moment, the temperature within the cooler box was lowered to approximately -0.1°C to -1.0°C, the lowering of the temperature was stopped, and then the shapes of the ice crystals were observed. The results of observance are shown in Figs. 2 to 8. In all the fish species used in the experiment, bi-pyramidal ice crystals were observed, confirming that all of these fish species had antifreeze proteins. Moreover, similar experiments were carried out using 1 µl of the suspension of fish paste and 1 µl of the suspension of dry foods. In these experiments, bi-pyramidal ice crystals were observed in all the fish species.

### Example 2

### (Separation and purification of antifreeze protein 1)

### a) Purification of antifreeze protein

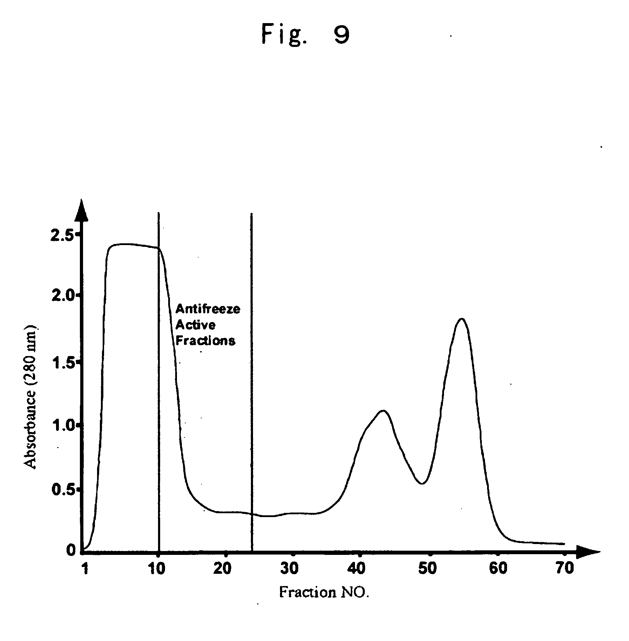

In a manner similar to that of Example 1(2)a), 30 ml of blood was collected from *Myoxocephalus stelleri* (Tilesius) in a plastic test tube. 30 ml of the blood of *Myoxocephalus stelleri* (Tilesius) collected in the plastic test tube was allowed to stand overnight within a refrigeration chamber at 4°C, so as to cause coagulation and precipitation (generally referred to as the step of clotting) of only a blood cell component to take place. Approximately a half (15 ml) of the 30 ml of blood was precipitated as blood cells, and then only 15 ml of the serum of the supernatant was sampled. At this time, centrifugation was carried out at 3000 revolutions for 15 minutes using a centrifugal machine for concentration, so as to improve the ability of separating blood cells from serum. At this time, bi-pyramidal ice crystals were observed in 15 ml of the sampled serum, confirming the presence of antifreeze proteins. This 15 ml of the serum was subjected to gel filtration by a SephadexG-50 gel column chromatography with an internal diameter of 2.5 cm and a height of 96 cm (capacity: 475 ml). A 0.1M ammonium sulfate aqueous solution (pH=7.9) was used as an effluent. Absorption patterns of 280-nm ultraviolet rays of the gel filtration fractions of the sera are shown in Fig. 9. 10 ml of a gel filtration fraction was obtained per test tube. Next, the fractions were frozen and dried.

0.5 to 2 ml of 0.1M ammonium sulfate aqueous solution (pH=7.9) was added to the frozen and dried products of each fraction, and then bi-pyramidal ice crystals in each solution were observed. Fraction numbers 10 to 24 (shaded part in Fig. 9: antifreeze Active Fractions) containing antifreeze proteins were used in the next purification step. Approximately 40 ml of an aqueous solution of proteins with a molecular weight of 30,000 or less containing antifreeze proteins was obtained from fraction numbers 10 to 24 dissolved in approximately 45 ml of an ammonium sulfate aqueous solution (pH=7.9) using Amicon Centriprep (Millipore corporation). To this solution, 5 ml of DEAE sepharose CL-6B anion exchange resin (manufactured by Pharmacia Corporation) equilibrated with 0.1 M ammonium sulfate aqueous solution (pH=7.9) was added. The solution was centrifuged using a centrifugal machine for concentration at 3000 revolutions for 1 minute, thereby obtaining approximately 40 ml of an aqueous solution of antifreeze proteins non-binding to the anion resin. To this solution, 5 ml of CM sepharose CL-6B cation exchange resin (manufactured by Pharmacia Corporation) equilibrated with a 0.1 M ammonium sulfate aqueous solution (pH=7.9) was added. The solution was centrifuged using a centrifugal machine for concentration at 3000 revolutions for 1 minute, thereby obtaining an aqueous solution of antifreeze proteins also non-binding to the cation exchange resin.

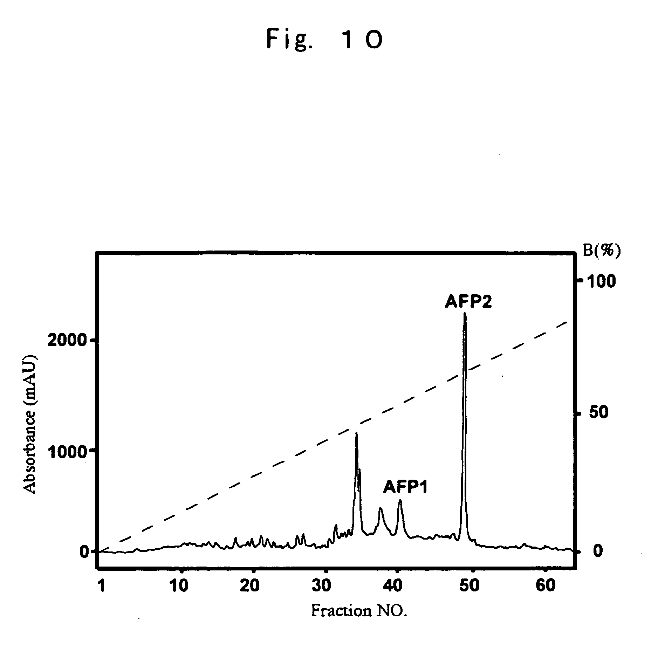

This sample was frozen, dried, and dissolved in a small volume of 0.1 M ammonium sulfate aqueous solution (pH=7.9), and then fractionated by reverse phase HPLC chromatography using an ODS (C8) column. Equilibration was carried out within the pathway of HPLC and the column using a 0.1 % trifluoroacetic acid aqueous solution (liquid A), and elution was carried out using acetonitrile containing 0.1% trifluoroacetic acid (liquid B). The concentration gradient of the liquid B was set so that the concentration was linearly elevated by 1% every minute, and the liquid flow rate was set at 1 ml/min. The patterns obtained by this reverse phase HPLC are shown in Fig. 10. Peak fractions observed in Fig. 10 were separately collected, frozen, and dried. Each of the products was dissolved in a small volume of a 0.1M ammonium sulfate aqueous solution (pH=7.9), and then the presence or the absence of the generation of bi-pyramidal ice crystals was tested. As a result, it was shown that AFP1 generated an ice crystal of dodecahedrons having irregular tetragons shown in Fig. 1B, while AFP2 generated an ice crystal in the shape of a dihexahedron shown in Fig. 1A. Hence, frozen and dried products of 2 peak fractions marked with AFP1 and AFP2 were obtained as purified antifreeze protein powder.

It was also confirmed that AFP1 and AFP2 were 2 different types of antifreeze proteins by electrophores as shown below.

### b) Gel electrophoresis

AFP1 and AFP2 derived from *Myoxocephalus stelleri* (Tilesius) were subjected to SDS gel electrophoresis. Since the molecular weights of AFP1 and AFP were predicted to be between approximately several thousands to 5 million, 16% SDS concentration in gel was selected so that proteins could be separated well by electrophoresis. Compositions of reagents used for electrophoresis are as follows.

| Separation gel (10 ml) | |
|---|---|
| 2.0 ml | Water |
| 5.4 ml | 30% polyacrylamide + 0.8% bis-acrylamide |
| 2.5 ml | 1.5M Tris-hydrochloric acid (pH=8.8) |
| 120 µl | 10% sodium dodecylsulfate solution |
| 70 µl | 10% ammonium persulfate solution |
| 15.0 µl | N, N, N', N'-tetramethylenediamine solution |

| Concentration gel (5 ml) | |
|---|---|
| 3.17 ml | Water |
| 500 µl | 30% polyacrylamide +0.8% bis-acrylamide |
| 1.25 ml | 0.5M Tris-hydrochloric acid (pH=6.8) |
| 50 µl | 10% sodium dodecylsulfate solution |
| 30 µl | 10% ammonium persulfate solution |
| 7.5 µl | N, N, N', N' tetramethylenediamine solution |

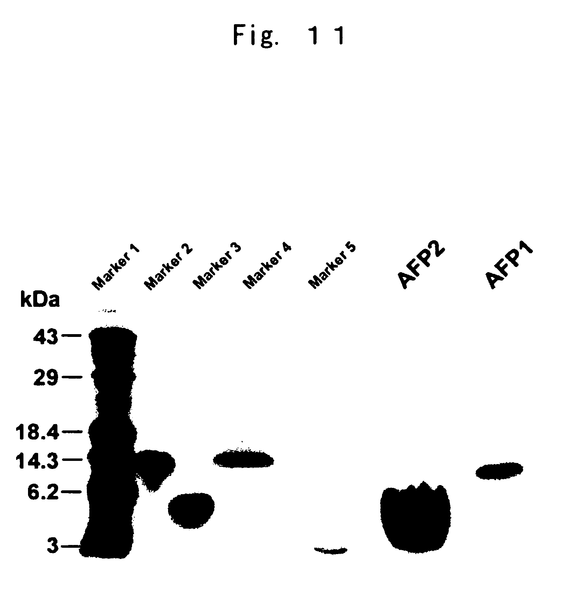

As a molecular weight marker (Fig. 11, Marker 1), a standard marker (manufactured by Gibco) for low-molecular-weight proteins was used. Moreover, for size comparison with proteins of the same type, a dimeric antifreeze protein RD3 (molecular weight of 14 kDa, Marker 2), a monomeric antifreeze protein RD3N1 (6.5 kDa, Marker 3), fowl eggwhite lysozyme (molecular weight of 14.3 kDa, Marker 4), and an antifreeze glycoprotein (3 kDa, Marker 4) were used. Each of all the proteins other than the markers was dissolved in 7 µl of a 0.1 M ammonium sulfate aqueous solution (pH=7.9). To this solution, 7 µl of a buffer solution (0.065 M Tris-HCl (pH=6.8), 2% SDS, 10% sucrose, 5% β-mercapthoethanol, 0.001% BPB staining reagent) was added, and the resultant was boiled at 95°C for 5 minutes, thereby preparing a sample for electrophoresis. The sample was subjected to 2 hours of electrophoresis under a constant voltage of 100 V using a mini slab gel electrophoresis system (manufactured by Nihon Eido Co., Ltd.). The results are shown in Fig. 11. As shown in this figure, AFP1 was an antifreeze protein with an approximate molecular weight of 11 kDa, and AFP2 was an antifreeze protein with a molecular weight of 3 to 6 kDa. Based on these molecular weights, it is inferred that AFP1 belongs to the above type IV (AFPIV), and AFP 2 belongs to type I (AFPI).

### Example 3

### (Separation and purification of antifreeze protein 2)

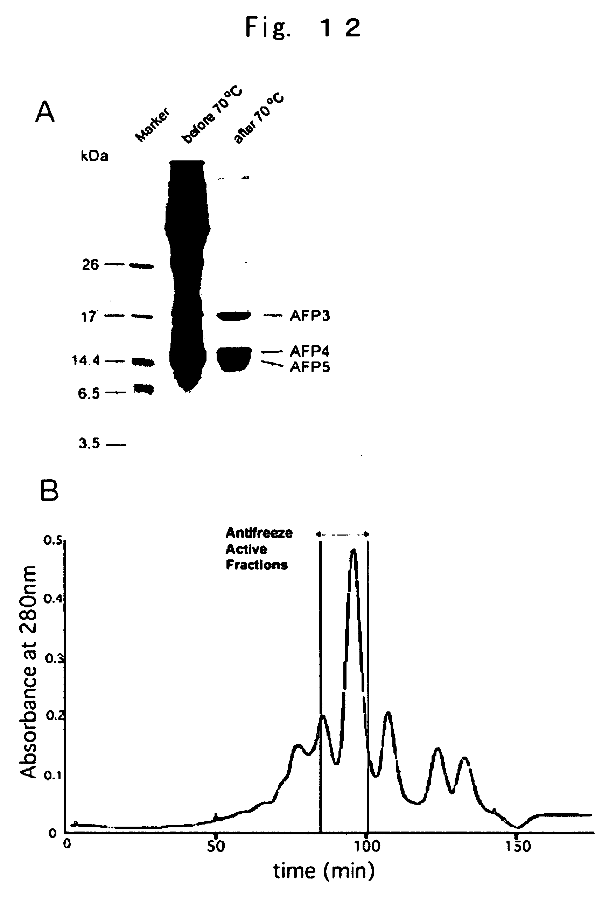

According to the procedures described in Example 1(2), 40 ml of a suspension of fish paste of *Hypomesus pretiosus japonicus* (Brevoort) was prepared. This suspension was put into a plastic test tube and centrifuged at 10,000 revolutions for 15 minutes, thereby obtaining approximately 20 ml of a supernatant solution. The protein concentration in the supernatant solution was approximately 25 mg/ml. Heat treatment was carried out by soaking the plastic test tube containing the supernatant solution in a warm bath at 70°C for 10 minutes. The plastic test tube was removed from the warm bath and then rapidly cooled by being inserted into crushed ice. Subsequently, the sample was centrifuged at 18,000 revolutions for 30 minutes, thereby obtaining approximately 15 ml of a supernatant solution. The protein concentration in this supernatant solution was approximately 6.6 mg/ml. The results of SDS gel electrophoresis carried out for the supernatant solution (Fig. 12A) and absorption patterns of ultraviolet rays obtained by gel column chromatography (Fig. 12B) are shown in Fig. 12 (Figure showing the results of electrophoresis). SDS gel electrophoresis was carried out under the same conditions as those in Fig. 9, and then gel column chromatography was carried out by eluting 200 µl of the sample solution at 0.2 ml/min using an Amersham Pharmacia Biotech Sepharose 6 HR10/30 column. All of AFP3 (approximate molecular weight of 17 kDa), AFP4, and AFP5 (approximate molecular weight of 14 kDa) shown in the right-most lane in Fig. 12A were antifreeze proteins derived from *Hypomesus pretiosus japonicus* (Brevoort) generating bi-pyramidal ice crystals in the shape shown in Fig. 1A. Based on these molecular weights, it was inferred that all the antifreeze proteins derived from *Hypomesus pretiosus japonicus* (Brevoort) belong to type II. Fractions of gel chromatography corresponding to AFP3, AFP4, and AFP5 correspond to portions within the range indicated with an arrow in Fig. 12B. The absorption peak intensity of this portion was compared with the remaining portion, and the centrifugal supernatant after 10 minutes of heat treatment at 70°C was obtained, showing that the antifreeze proteins derived from *Hypomesus pretiosus japonicus* (Brevoort) can be purified with a purification degree of approximately 62%.

The suspension of fish paste obtained from *Myoxocephalus stelleri* (Tilesius) was also subjected to separation and purification of antifreeze proteins, including a heat treatment process and gel electrophoresis, in a manner similar to the above procedures. In this case, antifreeze proteins thought to be of type I were mainly obtained. This antifreeze protein could also be obtained by heating to 90°C.

### Example 4

### (Separation and purification of antifreeze protein 3)

According to the procedures described in Example 1(2), 40 ml of a suspension of dry foods of *Spirinchus lanceolatus* (Hikita) was collected in a plastic test tube. 0.4 ml of 99.5% acetic acid was added to the suspension, mixed well, and then allowed to stand at 4°C for 1 hour. An acidic precipitate generated by this procedure was removed by centrifugation at 18,000 revolutions for 30 minutes, thereby obtaining approximately 35 ml of a supernatant solution. To this solution, 0.5 ml of 1 M sodium hydroxide aqueous solution was added, mixed well, and then allowed to stand at 4°C for 1 hour. A basic precipitate generated by this procedure was removed by centrifugation at 18,000 revolutions for 30 minutes, thereby obtaining approximately 32 ml of a supernatant solution. This solution was subjected to the separation and purification of antifreeze proteins, including a heat treatment process similarly to the above procedures, so that antifreeze proteins thought to be of type II were obtained.

### Example 5

### (Separation and purification of antifreeze protein 4)

According to the procedures described in Example 1(2), 40 ml of a suspension of fish paste of *Zoarces elongatus* (Kner) was prepared. The suspension was put into a plastic test tube and centrifuged at 6,000 revolutions for 30 minutes, thereby obtaining approximately 20 ml of a supernatant solution. The supernatant solution was dialyzed against a 50 mM sodium acetate buffer (pH=3.7), so that contaminating proteins were aggregated. This was removed by centrifugation at 12,000 revolutions for 30 minutes, thereby obtaining a supernatant solution. The supernatant solution was subjected to cation exchange chromatography, and then 1 ml of each eluate was collected using a fraction collector, while detecting absorbance at 280 nm. For cation exchange chromatography, an FPLC system of Amersham Pharmacia Biotech, and a High-S column of BIO-RAD were used. Column equilibration and incorporation of the supernatant solution were carried out using a 50 mM sodium acetate buffer (pH=3.7). Elution was carried out by applying a linear gradient of 0 to 0.5 M sodium chloride at a flow rate of 1 ml/minute. All the procedures upto this point were carried out at 4°C. Next, a sample solution for which absorbance at 280 nm was observed was purified by reverse phase chromatography using a TOSO HPLC system and an ODS column. Column equilibration and incorporation of the sample solution were carried out using 0.1% trifluoroacetic acid, and elution was carried out using an acetonitrile linear gradient. The absorbance of the elution sample was detected at 214 nm and 280 nm. The eluate fraction containing a single protein was obtained, frozen, and then dried. The frozen and dried powder was dissolved in a 0.1 M ammonium bicarbonate aqueous solution, and bi-pyramidal ice crystals in the solution were observed, thereby confirming that the proteins were antifreeze proteins. The sample of the antifreeze proteins was digested with Aspaginylendopeptidase and Trypsin. Peptide fragments obtained with each of these enzymes were collected again by reverse phase chromatography. Amino acid sequences of these peptide fragments were determined using an amino acid sequencer composed of a 491 Protein Sequencer, 785A Programmable Absorbance Detector, and 140C Microgradient System (manufactured by Applied Biosystems). According to the results, the antifreeze protein derived from *Zoarces elongatus* (Kner) contained 3 types of proteins comprising 65, 66, and 67 residues, respectively.

The amino acid sequences of these 3 types of antifreeze proteins and the nucleotide sequences of CDS regions of the genes thereof are respectively shown in SEQ ID NOS: 1 to 6 in the sequence listing. These nucleotide sequences show approximately 75% to 90% homology with that of the type III antifreeze protein derived from *Macrozoarces americanus* (Block et Schneider), whose nucleotide sequence has already been shown. Thus, it was shown that the type III antifreeze protein was purified from the fish paste of *Zoarces elongatus* (Kner).

The purification method shown in the method of Example 1 was applied to blood, or the suspensions of fish paste or dry foods of fish species used in the present invention. Purification was carried out until a single band appeared upon SDS electrophoresis, and then identification of antifreeze protein types I to IV was carried out for the following fish species. The results are shown below.

### a) type I

*Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microstomus achne* (Jordan et Starks), *Lepidopsetta mochigarei* (Snyder), *Platichthys stellatus* (Pallas), *Kareius bicoloratus* (Basilewsky), and *Eopsetta grigorjewi* (Herzenstein)

### b) type II

*Hypomesus pretiosus japonicus* (Brevoort), *Hypomesus olidus* (Dallas), *Spirinchus lanceolatus* (Hikita), *Mallotus villosus* (Muller), *Clupea pallasii* (Valenciennes), *Trachurus japonicus* (Temminck et Schlegel), and *Brachyopsis rostratus* (Tilesius)

### c) type III

*Zoarces elongatus* (Kner), *Ascoldia variegata knipowitschi* (Soldatov), *Pholidapous dybowskii* (Steindachner), *Pholis picta* (Kner), and *Opisthocentrus ocellatus* (Tilesius)

### d) type IV

*Myoxocephalus stelleri* (Tilesius), *Gymnocanthus herzensteini* (Jordan et Starks), *Myoxocephalus polyacanthocephalus* (Pallas), *Hemilepidotus jordani* (Bean), and *Furcina osimae* (Jordan et Starks)

(Note: These fish express both I and IV types)

### e) AFGP

*Gadus macrocephalus* (Tilesius) and *Theragra chalcogramma* (Pallas)

All publications, patents and patent applications cited herein are incorporated herein by reference in their entirety.

### Industrial Applicability

As described above, antifreeze proteins can prevent deterioration of food flavor and tissue disruption due to the growth of ice crystals in, for example, ice cream and frozen foods. Moreover, the antifreeze protein is expected to be an effective additive that can cancel the blocking of piping systems by recrystallization of ice such as in a cold heat supply system or a cold heat storage using ice slurries. Furthermore, the antifreeze protein is a promising substance for long-term low-temperature storage, or frozen storage of oocytes, sperm, transplants, and the like.

However in current circumstances, highly effective antifreeze proteins have been found only in specific fish species inhabiting polar areas, making it impossible to produce the protein in large quantities and thus obstructing the effective use thereof. In contrast, the new antifreeze protein of the present invention can be produced from fish species inhabiting water areas in Japan, water areas close to Japan, and water areas wherein the climate is equivalent to that of the above water areas. Thus, the antifreeze protein of the present invention can be easily obtained and can greatly contribute to the promotion of the use of antifreeze proteins and the development of studies on the application of antifreeze proteins.

### Sequence Listing Free Text

SEQ ID NO: 1: amino acid sequence of antifreeze protein derived from *Zoarces elongatus* (Kner)
SEQ ID NO: 2: nucleotide sequence of antifreeze protein derived from *Zoarces elongatus* (Kner)
SEQ ID NO: 3: amino acid sequence of antifreeze protein derived from *Zoarces elongatus* (Kner)
SEQ ID NO: 4: nucleotide sequence of antifreeze protein derived from *Zoarces elongatus* (Kner)
SEQ ID NO: 5: amino acid sequence of antifreeze protein derived from *Zoarces elongatus* (Kner)
SEQ ID NO: 6: nucleotide sequence of antifreeze protein derived from *Zoarces elongatus* (Kner)

## Claims

1. A method of collecting antifreeze glycoprotein, type I antifreeze protein, type II antifreeze protein, type III antifreeze protein, or type IV antifreeze protein while removing the odors of fish bodies that are the source from which antifreeze proteins are obtained, which comprises the following steps 1) to 5):
1) preparing a suspension of a paste composed of fish bodies or crushed dry food composed of fish bodies;
2) centrifuging the suspension of the paste composed of fish bodies or the crushed dry food composed of fish bodies obtained in 1) to obtain a supernatant solution;
3) carrying out heat treatment within a temperature range between 50°C and 70°C, or between 70°C and 98°C, of the supernatant solution obtained in 2);
4) obtaining a supernatant solution containing antifreeze proteins by removing the precipitate generated in 3) by centrifugation; and
5) collecting the antifreeze proteins from the supernatant solution obtained in 4).

2. A method according to claim 1, wherein said fish bodies are of fish species belonging to the genus *Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, Pholidapus,* or *Etrumeus,* where such fish species inhabit the water areas in Japan or the peripheral water areas of Japan, or water areas wherein the climate is equivalent to that of the above water areas.

3. A method according to claim 2, wherein the fish bodies are of *Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Hypomesus pretiosus japonicus* (Brevoort), *Hypomesus olidus* (Dallas), *Spirinchus lanceolatus* (Hikita), *Mallotus villosus* (Müller), *Pleurogrammus monopterygius* (Pallas), *Sebastes steindachneri* (Hilgendorf), *Cluped pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Eopsetta grigorjewi* (Herzenstein), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), or *Pholidapous dybowskii* (Steindachner).

4. A method according to claim 3, wherein when the fish bodies are of *Myoxocephalus stelleri* (Tilesius), *Gymnocanthus herzensteini* (Jordan et Starks), *Myoxocephalus polyacanthocephalus* (Pallas), *Hemilepidotus jordani* (Bean) or *Furcina osimae* (Jordan et Starks), the heat treatment of step (3) is carried out within a temperature range of between 70° and 98° C;
and wherein when the fish bodies are of *Hypomesus pretiosus japonicus* (Brevoort), *Hypomesus olidus* (Dallas), *Spirinchus lanceolatus* (Hikita), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), or *Pholidapous dybowsldi* (Steindachner), the heat treatment of step (3) is carried out within a temperature range of between 50° and 70° C.

## Patentansprüche

1. Verfahren zum Gewinnen von Anti-Frost-Glykoprotein, Typ-I-Anti-Frost-Protein, Typ-II-Anti-Frost-Protein, Typ-III-Anti-Frost-Protein oder Typ-IV-Anti-Frost-Protein während des Entfernens der Gerüche von Fischkörpern, welche die Quelle sind, von denen Anti-Frost-Proteine gewonnen werden, das die folgenden Schritte 1) bis 5) umfasst:
1) Herstellen einer Suspension einer Paste, die aus Fischkörpern oder aus zerstoßener Trockennahrung aus Fischkörpern zusammengesetzt ist;
2) Zentrifugieren der Suspension der Paste, die aus Fischkörpern oder der zerstoßenen Trockennahrung aus Fischkörpern zusammengesetzt ist und in 1) erhalten wurde, um eine Überstand-Lösung zu erhalten;
3) Ausführen von Hitzebehandlung der Überstand-Lösung, die in Schritt 2) erhalten wurde, in einem Temperaturbereich zwischen 50 °C und 70 °C oder zwischen 70 °C und 98 °C;
4) Erhalten einer Anti-Frost-Proteine enthaltenden Überstand-Lösung durch Entfernen des Niederschlags, der in Schritt 3) erzeugt wurde, mittels Zentrifugieren;
5) Gewinnen der Anti-Frost-Proteine aus der Überstand-Lösung, die in Schritt 4) erhalten wurde.

2. Verfahren nach Anspruch 1, worin die Fischkörper von Fischarten stammen, die zur Gattung Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, Pholidapus oder Etrumeus gehören, wo derartige Fischarten in den Wassergebieten Japans oder in den peripheren Wassergebieten Japans oder in Wassergebieten, wo das Klima jenem der oben genannten Wassergebiete entspricht, leben.

3. Verfahren nach Anspruch 2, worin die Fischkörper von Myoxocephalus stelleri (Tilesius), Gymnocanthus herzensteini (Jordan et Starks), Myoxocephalus polyacanthocephalus (Pallas), Hemilepidotus jordani (Bean), Furcina osimae (Jordan et Starks), Hypomesus pretiosus japonicus (Brevoort), Hypomesus olidus (Dallas), Spirinchus lanceolatus (Hikita), Mallotus villosus (Müller), Pleurogrammus monopterygius (Pallas), Sebastes steindachneri (Hilgendorf), Clupea pallasii (Valenciennes), Limanda herzensteini (Jordan et Snyder), Limanda punctatissima (Steindachner), Limanda schrenki (Schmidt), Limanda aspera (Pallas), Liopsetta obscura (Herzenstein), Clidoderma asperrimum (Temminck et Schlegel), Cleisthenes pinetorum herzensteini (Schmidt), Microctomus achne (Jordan et Starks), Lepidopsetta mochigarei (Snyder), Platichthys stellatus (Pallas), Kareius bicoloratus (Basilewsky), Eopsetta grigorjewi (Herzenstein), Gadus macrocephalus (Tilesius), Theragra chalcogramma (Pallas), Ammodytes personatus (Girard), Hypoptychus dybowskii (Steindachner), Trachurus japonicus (Temminck et Schlegel), Brachyopsis rostratus (Tilesius), Pholis picta (Kner), Opisthocentrus ocellatus (Tilesius), Zoarces elongatus (Kner), Ascoldia variegata knipowitschi (Soldatov) oder Pholidapous dybowskii (Steindachner) stammen.

4. Verfahren nach Anspruch 3, worin die Hitzebehandlung in Schritt 3) in einem Temperaturbereich zwischen 70 °C und 98 °C ausgefüh rt wird, wenn die Fischkörper von Myoxocephalus stelleri (Tilesius), Gymnocanthus herzensteini (Jordan et Starks), Myoxocephalus polyacanthocephalus (Pallas), Hemilepidotus jordani (Bean) oder Furcina osimae (Jordan et Starks) stammen;
und worin die Hitzebehandlung in Schritt 3) in einem Temperaturbereich zwischen 50 ° und 70 °C ausgeführt wird, wenn die Fischkörpe r von Hypomesus pretiosus japonicus (Brevoort), Hypomesus olidus (Dallas), Spirinchus lanceolatus (Hikita), Pleurogrammus monopterygius (Pallas), Sebastes steindachneri (Hilgendorf), Clupea pallasii (Valenciennes), Limanda herzensteini (Jordan et Snyder), Limanda punctatissima (Steindachner), Limanda schrenki (Schmidt), Limanda aspera (Pallas), Liopsetta obscura (Herzenstein), Clidoderma asperrimum (Temminck et Schlegel), Cleisthenes pinetorum herzensteini (Schmidt), Microctomus achne (Jordan et Starks), Lepidopsetta mochigarei (Snyder), Platichthys stellatus (Pallas), Kareius bicoloratus (Basilewsky), Gadus macrocephalus (Tilesius), Theragra chalcogramma (Pallas), Ammodytes personatus (Girard), Hypoptychus dybowskii (Steindachner), Trachurus japonicus (Temminck et Schlegel), Brachyopsis rostratus (Tilesius), Pholis picta (Kner), Opisthocentrus ocellatus (Tilesius), Zoarces elongatus (Kner), Ascoldia variegata knipowitschi (Soldatov) oder Pholidapous dybowskii (Steindachner) stammen.

## Revendications

1. Procédé pour collecter une glycoprotéine antigel, une protéine antigel de type I, une protéine antigel de type II, une protéine antigel de type III, ou une protéine antigel de type IV, tout en éliminant les odeurs des corps des poissons qui sont la source d'obtention des protéines antigels, qui comprend les étapes 1) à 5) suivantes :
1) préparer une suspension d'une pâte composée de corps de poissons ou de nourriture sèche broyée composée de corps de poissons ;
2) centrifuger la suspension de la pâte composée de corps de poissons ou la nourriture sèche broyée composée de corps de poissons obtenue en 1) afin d'obtenir une solution de surnageant ;
3) réaliser un traitement par la chaleur, dans une plage de températures comprises entre 50 °C et 70 °C, ou entre 70 °C et 98 °C, de la solution de surnageant obtenue en 2) ;
4) obtenir une solution de surnageant contenant des protéines antigel en éliminant le précipité généré en 3) par centrifugation ; et
5) collecter les protéines antigel de la solution de surnageant obtenue en 4).

2. Procédé selon la revendication 1, où lesdits corps de poissons proviennent des espèces de poissons appartenant au genre *Myoxocephalus, Gymnocanthus, Hemilepidotus, Furcina, Hypomesus, Spirinchus, Mallotus, Pleurogrammus, Sebastes, Clupea, Limanda, Liopsetta, Clidoderma, Cleisthenes, Microstomus, Lepidopsetta, Platichthys, Kareius, Eopsetta, Gadus, Theragra, Ammodytes, Hypoptychus, Trachurus, Brachyopsis, Pholis, Opisthocentrus, Zoarces, Ascoldia, Pholidapus*, ou *Etrumeus,* où de telles espèces de poissons vivent dans les eaux du Japon ou dans les eaux périphériques du Japon, ou dans des eaux où le climat est équivalent à celui des eaux ci-dessus.

3. Procédé selon la revendication 2, où les corps de poissons proviennent de *Myoxocephalus stelleri* (Tilesius), *Gymnocanthus herzensteini* (Jordan et Starks), *Myoxocephalus polyacanthocephalus* (Pallas), *Hemilepidotus jordani* (Bean), *Furcina osimae* (Jordan et Starks), *Hypomesus pretiosus japonicus* (Brevoort), *Hypomesus olidus* (Dallas), *Spirinchus lanceolatus* (Hikita), *Mallotus villosus* (Müller), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Eopsetta grigorjewi* (Herzenstein), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), ou *Pholidapous dybowskii* (Steindachner).

4. Procédé selon la revendication 3, où lorsque les corps de poissons proviennent de *Myoxocephalus stelleri* (Tilesius), *Gymnocanthus herzensteini* (Jordan et Starks), *Myoxocephalus polyacanthocephalus* (Pallas), *Hemilepidotus jordani* (Bean) ou *Furcina osimae* (Jordan et Starks), le traitement par la chaleur de l'étape (3) est réalisé dans une plage de températures comprises entre 70 ° et 98 °C ;
et lorsque les corps de poissons proviennent de *Hypomesus pretiosus japonicus* (Brevoort), *Hypomesus olidus* (Dallas), *Spirinchus lanceolatus* (Hikita), *Pleurogrammus monopterygius* (Pallas), *Sebastes steindachneri* (Hilgendorf), *Clupea pallasii* (Valenciennes), *Limanda herzensteini* (Jordan et Snyder), *Limanda punctatissima* (Steindachner), *Limanda schrenki* (Schmidt), *Limanda aspera* (Pallas), *Liopsetta obscura* (Herzenstein), *Clidoderma asperrimum* (Temminck et Schlegel), *Cleisthenes pinetorum herzensteini* (Schmidt), *Microctomus achne* (Jordan et Starks), *Lepidopsetta mochigarei* (Snyder), *Platichthys stellatus* (Pallas), *Kareius bicoloratus* (Basilewsky), *Gadus macrocephalus* (Tilesius), *Theragra chalcogramma* (Pallas), *Ammodytes personatus* (Girard), *Hypoptychus dybowskii* (Steindachner), *Trachurus japonicus* (Temminck et Schlegel), *Brachyopsis rostratus* (Tilesius), *Pholis picta* (Kner), *Opisthocentrus ocellatus* (Tilesius), *Zoarces elongatus* (Kner), *Ascoldia variegata knipowitschi* (Soldatov), ou *Pholidapous dybowskii* (Steindachner), le traitement par la chaleur de l'étape (3) est réalisé dans une plage de températures comprises entre 50 ° et 70 °C.


### SEQUENCE LISTING

<110> National Institute of Advanced Industrial Science and Technology
<120> Anti-freeze protein from fish
<130> PH-1675-PCT
<140>
   <141>
<150> JP 2001-356709
   <151> 2001-11-21
<150> JP 2002-104477
   <151> 2002-04-05
<150> JP 2002-192339
   <151> 2002-07-01
<150> JP 2002-320425
   <151> 2002-11-01
<160> 6
<170> PatentIn Ver. 2. 1
<210> 1
   <211> 65
<212> PRT
   <213> Zoarces elongatus Kner
<400> 1
   Gly Gln Ser Val Val Ala Thr Gln Leu Ile Pro Met Asn Thr Ala Leu
   1 5 10 15
   Thr Pro Ala Met Met Glu Gly Lys Val Thr Asn Pro Ser Gly Ile Pro
   20 25 30
   Phe Val Glu Ala Ser Gln Ile Val Gly Lys Gln Val Asn Val Ile Val
   35 40 45
   Ala Lys Gly Gln Thr Leu Met Pro Asp Met Val Lys Thr Tyr Ala Gly
   50 55 60
   Lys
   65
<210> 2
   <211> 195
   <212> DNA
   <213> Zoarces elongatus Kner
<220>
   <221> CDS
   <222> (1).. (195)
<400> 2
   ggc cag tcc gtg gtg gcc acc cag ctg atc ccc atg aat act gcc ctg    48
   Gly Gln Ser Val Val Ala Thr Gln Leu Ile Pro Met Asn Thr Ala Leu
   1               5                   10                  15
   act ccg gcg atg atg gag ggg aag gtg acc aac cca agc ggc atc ccg    96
   Thr Pro Ala Met Met Glu Gly Lys Val Thr Asn Pro Ser Gly Ile Pro
               20                  25                  30
   ttc gtg gag gcg tcc caa ata gtg ggg aag caa gtg aac gtg ata gtg    144
   Phe Val Glu Ala Ser Gln Ile Val Gly Lys Gln Val Asn Val Ile Val
           35                  40                  45
   gct aag ggc caa acc ctc atg cca gac atg gtg aaa acg tac gcg gga    192
   Ala Lys Gly Gln Thr Leu Met Pro Asp Met Val Lys Thr Tyr Ala Gly
       50                  55                  60
   aag                                                                195
   Lys
   65
<210> 3
   <211> 66
   <212> PRT
   <213> Zoarces elongatus Kner
<400> 3
   Asn Gln Glu Ser Val Val Ala Ala Val Leu Ile Pro Ile Asn Thr Ala
   1 5 10 15
   Leu Thr Val Gly Met Met Thr Thr Arg Val Val Ser Pro Thr Gly Ile
   20 25 30
   Pro Ala Glu Asp Ile Pro Arg Leu Ile Ser Met Gln Val Asn Gln Val
   35 40 45
   Val Pro Met Gly Thr Thr Leu Met Pro Asp Met Val Lys Gly Tyr Ala
   50 55 60
   Pro Ala
   65
<210> 4
   <211> 198
   <212> DNA
   <213> Zoarces elongatus Kner
<220>
   <221> CDS
   <222> (1).. (198)
<400> 4
   aac cag gag tcc gtg gtg gcc gcc gtt ctg atc ccc ata aat act gcc 48
   Asn Gln Glu Ser Val Val Ala Ala Val Leu Ile Pro Ile Asn Thr Ala
   1 5 10 15
   ctg act gtg ggg atg atg acg aca cgg gtg gtc tcc cca acg ggc atc 96
   Leu Thr Val Gly Met Met Thr Thr Arg Val Val Ser Pro Thr Gly Ile
   20 25 30
   ccc gcc cag gac att ccc cga tta atc tca atg caa gtg aac cag gta 144
   Pro Ala Gln Asp Ile Pro Arg Leu Ile Ser Met Gln Val Asn Gln Val
   35 40 45
   gtg ccg atg ggc aca acc ctc atg cca gac atg gtg aaa ggg tac gcc 192
   Val Pro Met Gly Thr Thr Leu Met Pro Asp Met Val Lys Gly Tyr Ala
   50 55 60
   ccg gct 198
   Pro Ala
   65
<210> 5
   <211> 67
   <212> PRT
   <213> Zoarces elongatus Kner
<400> 5
   Asn Gln Ala Ser Val Val Ala Thr Gln Leu Ile Pro Ile Asn Thr Ala
   1 5 10 15
   Leu Thr Leu Val Met Met Lys Ala Lys Val Ala Thr Pro Met Gly Ile
   20 25 30
   Pro Ala Glu Asp Ile Pro Arg Ile Val Ser Met Gln Val Asn Gln Ala
   35 40 45
   Val Ala Cys Gly Thr Thr Leu Met Pro Gly Met Val Lys Thr Tyr Thr
   50 55 60
   Pro Val Lys
   65
<210> 6
   <211> 201
   <212> DNA
   <213> Zoarces elongatus Kner
<220>
   <221> CDS
   <222> (1) .. (201)
<400> 6
   aac cag gcg tcc gtg gtg gcc acc cag ctg atc ccc ata aat act gcc 48
   Asn Gln Ala Ser Val Val Ala Thr Gln Leu Ile Pro Ile Asn Thr Ala
   1 5 10 15
   ctg act ctg gtg atg atg aag gcg aag gtg gcc acc cca atg ggc atc 96
   Leu Thr Leu Val Met Met Lys Ala Lys Val Ala Thr Pro Met Gly Ile
   20 25 30
   ccc gcc gag gac att ccc cga ata gtc tca atg caa gtg aac cag gca 144
   Pro Ala Glu Asp Ile Pro Arg Ile Val Ser Met Gln Val Asn Gln Ala
             35                  40                  45
   gtg gcg tgt ggc aca acc ctc atg cca ggc atg gtg aaa acg tac act   192
   Val Ala Cys Gly Thr Thr Leu Met Pro Gly Met Val Lys Thr Tyr Thr
         50                  55                  60
   cca gtg aag                                                       201
   Pro Val Lys
    65